# EUROPEAN PATENT SPECIFICATION

(11) **EP 1 292 826 B1**
(45) Date of publication and mention of the grant of the patent: **28.11.2007**
(21) Application number: 01934780.6
(22) Date of filing: 23.05.2001
(51) Int. Cl.: G01N 33/497, G01N 33/00

(54) **CALIBRATION METHOD COMPENSATING FOR THE CONCENTRATION OF WATER VAPOR**
DIE KONZENTRATION VON WASSERDAMPF KOMPENSIERENDES KALIBRATIONSVERFAHREN
PROCEDE DE CALIBRAGE PERMETTANT LA COMPENSATION DE CONCENTRATION DE VAPEUR D'EAU

(30) Priority: 29.05.2000 SE 0001987
(43) Date of publication of application: 19.03.2003
(73) Proprietor: Servotek AB, 232 91 Arlöv (SE)
(72) Inventor: OLSSON, Sven, Gunnar, S-232 91 Arlöv (SE); BRAUER, Stefan, S-227 62 Lund (SE); LINGE, Anders, S-244 36 Kävlinge (SE); NIININEN, Tarmo, S-243 32 Höör (SE)
(74) Representative: Karlsson, Leif Gunnar Börje
(86) International application number: PCT/SE2001/001152
(87) International publication number: WO 2001/092878

(56) References cited:
- EP-A1- 0 411 500
- WO-A1-98/20346
- SE-B- 445 145
- INGVAR E. SODAL ET AL.: 'A new method for accurate calibration of respiratory flowmeters' BIOMEDICAL SCIENCES INSTRUMENTATION vol. 13, 1977, pages 69 - 71, XP002945559

## Description

### Background of the invention

### Field of the invention

The invention relates to a method in calibrating or checking an apparatus for determining in a person's exhalation air the concentration of a specific substance, said apparatus comprising means for passing exhalation air along a free measuring length thereof; and means for supplying air of a predetermined composition to the measuring length to produce an air flow along said length.

An apparatus of this kind is described in WO-A-9820346.

In order that the apparatus can give a reliable test result also when used in the field for the police department's "flying" check of the soberness of drivers it is a requirement that the apparatus is accurately calibrated and can easily be recalibrated and checked at intervals during use.

### Description of the Prior Art

US-A-4,793,173 describes a process for calibrating a gas metering instrument. A test chamber is flushed with a flushing gas and subsequently a specified amount of calibrating substance is mixed with the flushing gas in the test chamber to form a calibrating gas of predetermined composition. The calibrating substance can be supplied as a gas or as a liquid which is vaporized in the test chamber.

When an apparatus of the kind referred to above is to be calibrated it is essential for the accuracy of the test result obtained by such apparatus that also the concentration of water vapor in the air flow through the housing is measured and that this is taken into account when calibrating or checking the apparatus.

### Brief Summary of the Invention

It is a primary object of the invention to provide a calibration method wherein also the concentration of water vapor is taken into account in order to improve the accuracy that is attained in calibrating the apparatus of the kind referred to.

This is achieved by the method of the kind referred to according to claim 1 being characterized in that a liquid mixture of said substance and water in a specified concentration thereof is instantaneously vaporized to be injected as a gas into said air when supplied to the measuring length, that the concentration of said substance and the concentration of water vapor in the air flow along the measuring length is determined, that the total gas flow and the gas flow along the measuring length are calculated to determine the substance gas flow and the water vapor flow, and that the ratio thereof is compared with said specified concentration of said mixture.

Further details of the method of the invention are defined in the dependent claims.

### Brief Description of the Drawing

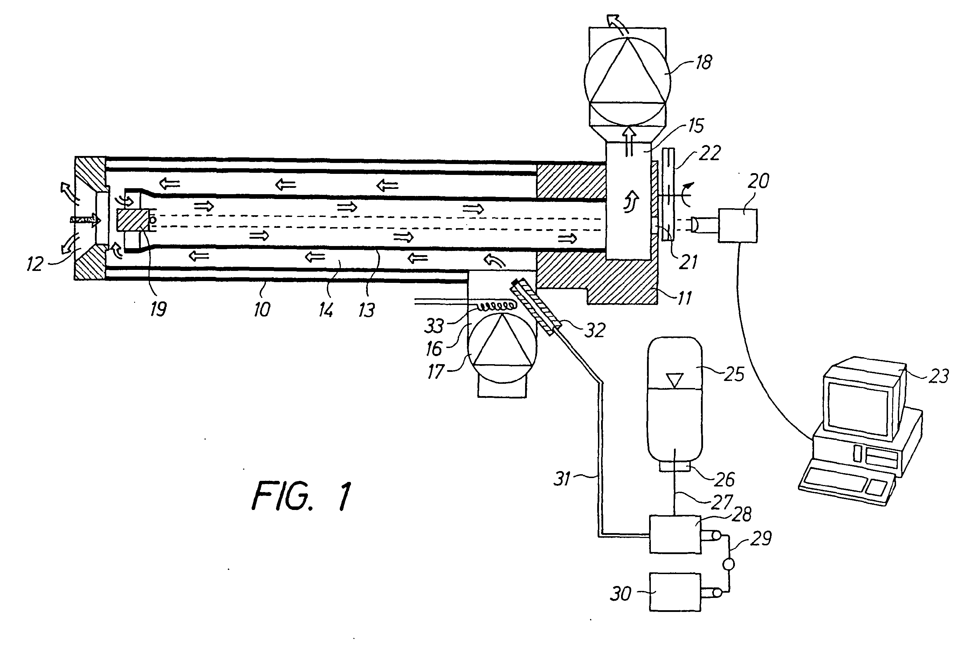

An illustrative embodiment of the method of the invention will be described in more detail below with reference to the accompanying drawing wherein
**FIG 1** is a diagrammatic axial cross sectional view of an apparatus on which the method of the invention can be applied said apparatus being of the kind disclosed in FIG 1 in WO-A-9820346,
**FIG 2** is a graph showing the measured content of the specific substance in the air flow over the time, and
**FIG 3** is a graph showing the measured content of water in the air flow over the time.

### Detailed Description of the Invention

The apparatus comprises a heat insulated cylindrical housing 10. The housing is closed at one end thereof by means of an end element 11 while it has an inlet opening 12 at the other end. A cylindrical cuvette 13 forming a measuring length is mounted coaxially inside the housing defining an annular passage 14 through the housing. An open end of the cuvette is axially spaced from the inlet opening 12, while the other end is attached to the end element 11 where it communicates with a transverse passage 15 in the end element. A conduit 16 having a fan 17 opens radially in the passage 14 adjacent the end element 11. A suction fan 18 is connected to the passage 15.

A radiation source (light source) 19 is supported in the open end of the cuvette 13, the radiation from said source being emitted towards a detector 20 through a window 21 and a filter wheel 22. A computer 23 is connected to the detector for processing the detector output signal.

When the apparatus described is used for measuring the concentration of a specific substance and the concentration of water in the exhalation air the fan 17 supplies a flow of air of predetermined composition to the passage 14. The air partly escapes to the ambient atmosphere through the opening 12 and partly passes through the cuvette 13 to the passage 15 and from there to the ambient atmosphere. The face of the person to be tested is flushed by the air escaping through the opening 12 before the person is exhaling into the inlet opening 12. Then, when exhalation takes place the exhalation air passes through the cuvette 13 and the concentrations of the specific substance and the water are measured by the detector 20. The fan 18 draws the exhalation air through the cuvette 13 and is used to control the flow through said cuvette particularly in case the person to be tested is unconscious. For further details of the testing method applied reference is made to WO-A-9820346.

For the purpose of the invention the apparatus is provided with a calibration or checking device including a closed container 25 containing a mixture of the substance for which the apparatus is to be calibrated, and water under overpressure. Said substance may be e.g. ethyl alcohol. The pressure may be established during filling of the container, or a flask with gas under pressure may be connected to the container. A membrane or plug 26 closes the container and can be sealed so that the content of the container cannot be manipulated or exchanged without breaking the seal. The membrane or plug is penetrated by a cannula 27 which connects the container with a valve 28 operatively connected by a suitable gear 29 to a solenoid 30 for operating the valve which is closed when the solenoid is deenergized. The valve should be a high speed valve having a very small dead space. A capillary tube 31 extends from the outlet of the valve 28 to a heated nozzle 32 which is located in the conduit 16 on the pressure side of the fan 17. In the conduit 16 there is also provided on the pressure side of the fan an electric heating coil 33.

During a calibration cycle air from a space wherein the air is free from the specific substance for which the apparatus shall be calibrated, or has a known concentration of said substance, is supplied by the fan 17 through the conduit 16 to create an air flow through the annular passage 14 towards the inlet opening 12. The air is heated by the electric heating coil 33 so that the surfaces in the apparatus defining the air passages therein are heated in order to avoid that humidity entrained in the air precipitates on said surfaces. A major part of the air supplied escapes to the ambient atmosphere through the inlet opening 12, and a minor part passes through the cuvette 13 into the passage 15 and via the fan 18 to the ambient atmosphere.

Liquid is supplied to the heated nozzle 32 from the container 25 by energizing the solenoid 30 and thus opening the valve 28. The flow of liquid is determined either by the overpressure in the container and the flow resistance in the capillary tube 31 the valve being held open continuously, or by operating the valve by short opening pulses in rapid succession. The liquid is vaporized in the heated nozzle 32 in order to be injected into the air flow as a gas the vaporization of the liquid being sustained by the heating effect of the heating coil 33. Condensing on the surfaces of the air passages is prevented by said surfaces being kept warm by the heated air flow.

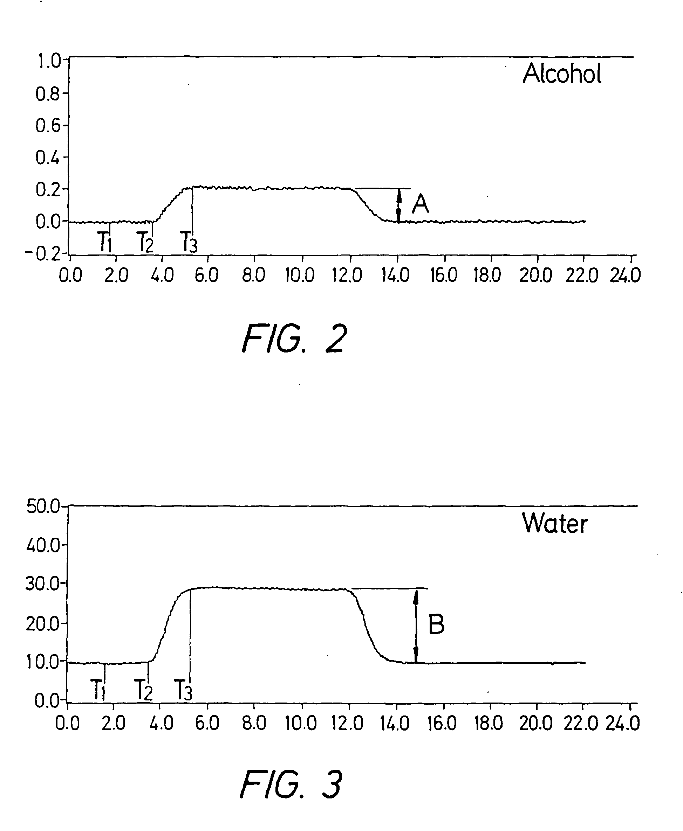

With reference to FIG 2 the injection of a predetermined volume of the liquid contained in the container 25 through the nozzle 33 is initiated a the time T₁. The content of the specific substance in the air flow as measured by the detector 20 will be zero until the air with the substance entrained therein reaches the left end of the cuvette 13 and passes into this cuvette at the time T₂. At this time the content of the specific substance as measured by the detector will increase and eventually will reach a maximum value at the time T₃. Since the volume of the flow passage from the injection site to the left end of the cuvette 13 and the volume of the flow passage in the cuvette 13 between the ends thereof are known as well as the time period between the times T₁ and T₂ and T₂ and T₃, respectively, the total gas flow and the flow through the cuvette 13 can be calculated. Since the volume of the injected liquid is also known the gas flow A of the specific substance (alcohol) can be calculated. All calculations are performed by the computer 23.

FIG 3 shows the corresponding graph for water in the air flow, and the flow B of water vapor is determined in the same manner.

Since the ratio A/B (concentration) of the liquid in the container 31 is known the apparatus can be calibrated to indicate the absolute values of the specific substance (alcohol) A and water B, respectively.

In the apparatus measurements can be made at (three) different wave lengths of the radiation emitted by the source 19 by adjustment of the filter wheel 22 in order to locate different filters therein in the radiated beam. When the apparatus is calibrated the graphs obtained at the different measurements are made congruent by adjustment in the computer. Then, when the apparatus is used for measuring unknown contents of a specific substance such as alcohol in exhalation air, it is guarantied that no other substance is involved if the results obtained at different wave lengths are the same. If the apparatus has been calibrated for ethyl alcohol and there is a divergence between the values in testing if the exhalation air of a person contains ethyl alcohol the divergence may be due to the fact that the exhalation air contains also e. g. methyl alcohol.

It is also possible to calibrate the apparatus for different substances so that discrimination between these substances can be made at testing.

The method of the invention can be used also for checking that an apparatus which has been calibrated by using the present method maintains the calibration, by using different containers 25 which contain different substances mixed with water and/or substance and water in different mixing ratios.

The invention has been described with reference to one specific embodiment of the apparatus disclosed in WO-A-9820346 but can applied also to the other embodiments described in WO-A-9820346 and to apparatuses in general that are constructed for practicing the method disclosed in WO-A-9820346.

## Claims

1. A method in calibrating or checking an apparatus for determining in a person's exhalation air the concentration of a specific substance, said apparatus comprising means for passing exhalation air along a free measuring length thereof, and means for supplying air of a predetermined composition to the measuring length to produce an air flow along said length **characterized in that** a liquid mixture of said substance and water in a specified concentration thereof is instantaneously vaporized to be injected as a gas into said air when supplied to the measuring length, that the concentration of said substance and the concentration of water vapor in the air flow along the measuring length is determined, that the total gas flow and the gas flow along the measuring length are calculated to determine the substance gas flow and the water vapor flow, and that the ratio thereof is compared with said specified concentration of said mixture.

2. The method of claim 1 wherein said substance is evaporated by being supplied to said air flow through a heated nozzle.

3. The method of claim 1 or 2 wherein said air flow is heated.

4. The method of any of claims 1 to 3 wherein the apparatus is calibrated for more than one specific substance.

## Patentansprüche

1. Verfahren zur Kalibrierung oder Überprüfung eines Apparates zur Bestimmung der Konzentration einer spezifischen Substanz in der Ausatemluft einer Person, wobei besagter Apparat Mittel umfasst, um die Ausatemluft entlang einer freien Messstrecke davon zu führen, und Mittel, um Luft mit einer vorgegebenen Zusammensetzung der Messstrecke zuzuführen, um einen Luftfluss entlang besagter Strecke zu erzeugen, **dadurch gekennzeichnet, dass** eine flüssige Mischung von besagter Substanz mit Wasser in einer spezifizierten Konzentration davon sofort verdampft wird, um als ein Gas in besagte Luft injiziert zu werden, wenn diese der Messstrecke zugeführt wird, dass die Konzentration von besagter Substanz und die Konzentration von Wasserdampf in dem Luftfluss entlang der Messstrecke bestimmt wird, dass der gesamte Gasfluss und der Gasfluss entlang der Messstrecke berechnet werden, um den Gasfluss der Substanz und den Wasserdampffluss zu bestimmen, und dass das Verhältnis davon mit besagter spezifizierter Konzentration von besagter Mischung verglichen wird.

2. Verfahren gemäß Anspruch 1, wobei besagte Substanz verdampft wird, indem sie dem besagten Luftfluss durch eine beheizte Düse zugeführt wird.

3. Verfahren gemäß Anspruch 1 oder 2, wobei besagter Luftfluss beheizt wird.

4. Verfahren gemäß einem der Ansprüche 1 bis 3, wobei der Apparat für mehr als eine spezifische Substanz kalibriert wird.

## Revendications

1. Procédé de calibrage ou de contrôle d'un appareil pour déterminer, dans l'air exhalé par une personne, la concentration d'une substance spécifique, ledit appareil comprenant des moyens pour amener l'air exhalé à passer le long d'une longueur de mesure libre de celui-ci, et des moyens pour diriger de l'air d'une composition prédéterminée vers la longueur de mesure dans le but de produire un écoulement d'air le long de ladite longueur, **caractérisé en ce qu'**un mélange liquide de ladite substance et d'eau dans une concentration spécifiée de celles-ci est instantanément vaporisé dans le but d'être injecté sous forme de gaz dans ledit air quand il est dirigé vers la longueur de mesure, **en ce que** la concentration de ladite substance et la concentration de vapeur d'eau dans l'écoulement d'air le long de la longueur de mesure est déterminée, **en ce que** l'écoulement de gaz total et l'écoulement de gaz le long de la longueur de mesure sont calculés dans le but de déterminer l'écoulement gazeux de la substance et l'écoulement de vapeur d'eau, et **en ce que** leur rapport est comparé à ladite concentration spécifiée dudit mélange.

2. Procédé selon la revendication 1, dans lequel ladite substance est évaporée en étant dirigée vers ledit écoulement d'air à travers une buse chauffée.

3. Procédé selon la revendication 1 ou 2, dans lequel ledit écoulement d'air est chauffé.

4. Procédé selon l'une quelconque des revendications 1 à 3, dans lequel l'appareil est calibré pour plus d'une substance spécifique.
